Europäisches Patentamt

European Patent Office

Office européen des brevets

(11) Publication number: **0 025 646**
**B1**

(12) **EUROPEAN PATENT SPECIFICATION**

(45) Date of publication of patent specification: **20.04.83**

(51) Int. Cl.³: **G 01 N 3/20, G 01 M 11/08**

(21) Application number: **80302845.5**

(22) Date of filing: **18.08.80**

(54) Method and apparatus for proof testing glass fibres.

(30) Priority: **20.09.79 GB 7932633**

(43) Date of publication of application:
**25.03.81 Bulletin 81/12**

(45) Publication of the grant of the patent:
**20.04.83 Bulletin 83/16**

(84) Designated Contracting States:
**AT BE CH DE FR GB IT LI NL SE**

(56) References cited:
**US - A - 2 496 029**

**IBM TECHNICAL DISCLOSURE BULLETIN, vol.
10, no. 4, September 1967 New York US
F. J. ABELING et al.: "Fiber optics flex tester",
pages 480—481**

**THE REVIEW OF SCIENTIFIC INSTRUMENTS,
vol. 44, no. 3, March 1973 New York US
M. J. SAUNDERS: "Optical Fiber breaking stress
distributions obtained by a cantilever method"
pages 330—333**

(73) Proprietor: **THE POST OFFICE
23 Howland Street
London W1P 6HQ (GB)**

(72) Inventor: **France, Paul William
Little Cottage
Newbourne Nr. Woodbridge Suffolk (GB)**

(74) Representative: **Smith, Norman Ian et al,
F.J. CLEVELAND & COMPANY 40-43 Chancery
Lane
London WC2A 1JQ (GB)**

(56) References cited:
**JOURNAL OF PHYSICS E: SCIENTIFIC
INSTRUMENTS, vol. 11, no. 2, 1978
London GB
L. A. JACKSON et al.: "A technique for
estimating the breaking strain of long lengths of
optical fibers" pages 161—165**

Courier Press, Leamington Spa, England

# Method and apparatus for proof testing glass fibers

This invention relates to testing glass fibres. In particular the inventon relates to the proof testing of optical glass fibres, to detect flaws in the glass and thus the suitability of the fibres for use as dielectric waveguides in optical communications systems.

Short gauge length tensile testing can yield much information concerning the flaw distribution in optical glass fibres of relatively short length but the information cannot be reliably extrapolated to the kilometer lengths necessary in optical fibre cables.

Proof testing is a way of more fully characterising long samples and can be used to ensure guaranteed strength for a given length of fibre, e.g. greater than that strength expected in subsequent cabling and service operations, and to ensure a minimum lifetime for a fibre in a stressed condition.

Convention proof testing apparatus operates by applying tensile strain to a fibre, which is wound between two capstans. One capstan is fitted with constant torque motor which determines the load on the fibre whilst the other drives the fibre at a constant speed.

This method can impart a strain of up to about 1% to the fibre, but suffers from the disadvantage that the tension necessary (about 600 grams) to impart this strain causes a considerable pressure between the fibre and the capstan and hence damage to the fibre surface even through its thin protective coating.

It is desirable to impart a high strain (above 1%) in the fibre without the risk of surface damage and the present invention aims to achieve this requirement.

According to a first aspect of the present invention there is provided a method of proof testing a glass fibre to detect fibres having a strength greater than a predetermined value, the method comprising moving the fibre along a path in which it is bent over the surface of a roller which is free to rotate, the roller having a diameter such that tensile strain induced by bending in the surface of said optical fibre corresponds to the said predetermined value of strength, the fibres being drawn around said roller so that successive portions of said fibre are subjected to the tensile strain induced by bending. Because the strain is induced by bending, the tensile load in the fibre can be very small (~30 gms) and hence high proof-test levels can be attained without damage to the fibre.

The tensile strain to which the surface of said fibre is subjected may be greater than 1%. The fibre may be passed through a set of three said rollers which are arranged such that said fibre is bent in a first sense about a first roller, then in a second sense opposite to said first sense about a second roller, and then in said first sense about a third roller. The rollers may be arranged so that the tensile strain induced by bending is maintained for substantially 90° about said first roller, for substantially 180° about said second roller, and for substantially 90° about said third roller.

The fibre may be passed through n sets of three rollers, the rollers of each set being orientated such that 2n axial lines on the surface of said fibre are subjected to a maximum value of said tensile strain induced by bending. n may be equal to 4, and the 4 sets of rollers located so that said 8 lines are equi-angularly spaced about the circumference of said fibre.

The fibre may be a plastics sheathed optical fibre.

According to a second aspect of the present invention there is provided apparatus for proof-testing glass fibres to detect glass fibres having a strength greater than a predetermined value, said apparatus comprising n sets of m rollers, where n and m are greater than 1, each roller being free to rotate and having a diameter such that tensile strain induced by bending in the surface of said optical fibre corresponds to the said predetermined value of strength, the rollers of each set of rollers being mounted in a respective one of n support means, said n support means being arranged at predetermined angles to each other, the arrangement being such that when a fibre is drawn through said sets of rollers maximum strain is induced in the fibre at positions substantially evenly distributed about the circumference of said fibre.

The apparatus may have three rollers and the support means may be a block, the rollers being mounted so that the axes of two rollers lie in a plane and are separated by a gap, the third roller being mounted with its axis parallel to, but out of said plane, and substantially midway between two planes which pass one through each axis of said two rollers in a direction normal to said one plane.

The apparatus may have a support means comprising a U-shaped block and the rollers may be located in the gap between the limbs of the block, each roller having a bearing means in each limb of the block.

Means for holding the blocks may comprise an open ended box having channels on its inner wall for receiving and holding said U-shaped blocks in predetermined orientations.

The invention will be described now by way of example only with particular reference to the accompanying drawings in which:—

Figure 1 shows a part sectional view of an optical glass fibre being pulled around a roller;

Figure 2 shows a section along the line X—X of Figure 1;

Figure 3 shows a sectional view of a set of three proof testing rollers as used in accordance with the invention;

**0 025 646**

Figure 4 illustrates in section an optical glass fibre tested in accordance with the invention;

Figure 5 shows a set of three rollers mounted in a block in accordance with the invention;

Figure 6 is a perspective view of a proof testing apparatus having four sets of three rollers, each set mounted at 45° to two other sets, and

Figure 7 is a perspective view of an alternative form of proof testing apparatus in accordance with the present invention.

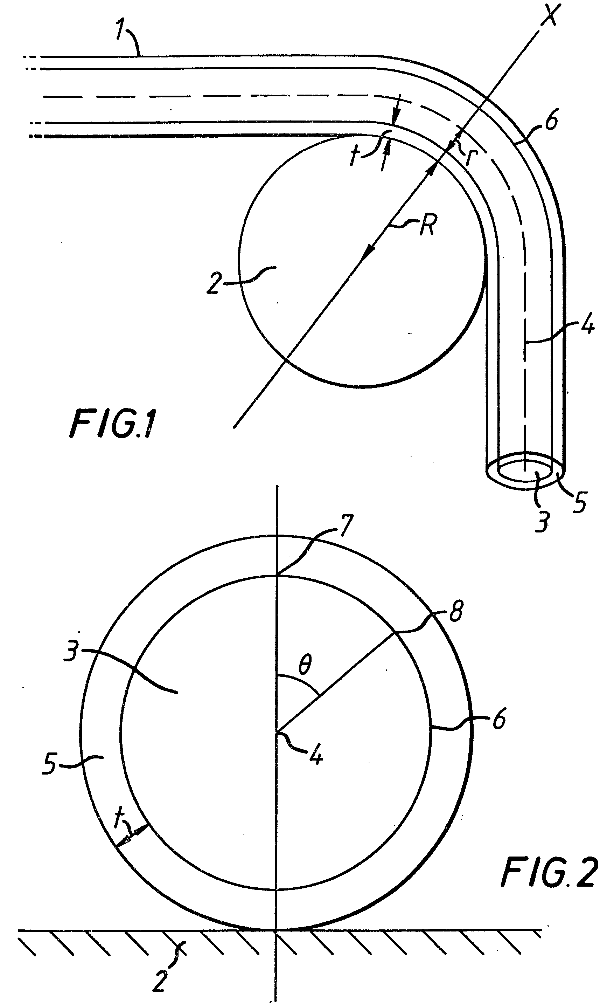

The present technique will be described initially with reference to Figure 1. In this Figure a plastics coated optical fibre 1 is shown being pulled along a path which passes round part of the circumference of a roller 2 of radius R. The plastics coated fibre 1 comprises a central glass portion 3 having a generally circular cross-section of radius and an outer protective coating 5 of thickness t. The fibre 1 is pulled around the roller 2 under sufficient tension to ensure a good contact between the plastics coated fibre 1 and the roller 2 for about a quarter of a revolution of the roller 2. The axis 4 of the fibre 1 can be seen to be curved into a circular path whose radius is R + t + r whilst the outer surface of the central glass portion 3 is under strain being curved into a path of R + t + 2r. As the fibre 1 is passed around the roller 2 the central glass portion 3 is progressively subjected to a strain whose maximum can be shown to be:

$$T\ max = \frac{r}{R+t+r} \times 100\%$$

The strain imparted to a fibre 1 by the roller 2 is therefore inversely proportional to the radius R of the roller 2 and the strain to which a fibre is to be proof tested determines the dimension of the roller to be used.

Referring now to Figure 2 it will be appreciated that the strain imparted varies with position within the central glass portion 3. The maximum strain T max occurs only along an axial line through the point 7 at a radially outermost position on the surface 6 of the glass portion 3. On any other axial line such as that through the point 8 on the surface of the glass portion 3 the strain will be $T = T\ max\ Cos\ \theta$, where $\theta$ is the angle between a radius to the point 7 and a radius to the point 8. The strain within the glass portion 3 will be proportional to its radius at that point. In the present technique a fibre is proof tested by pulling it along a path which extends around a roller which is free to rotate as shown in Figure 1. The diameter of the roller is such that the tensile strain induced by bending in the surface of the optical fibre corresponds to a predetermined value of strength which the fibre is required to have. It may be thought disadvantageous that only points on the surface of the glass are subjected to maximum strain but as the majority of fibre flaws are Griffith microcracks which lie on or near the surface, the technique will detect the majority of flaws.

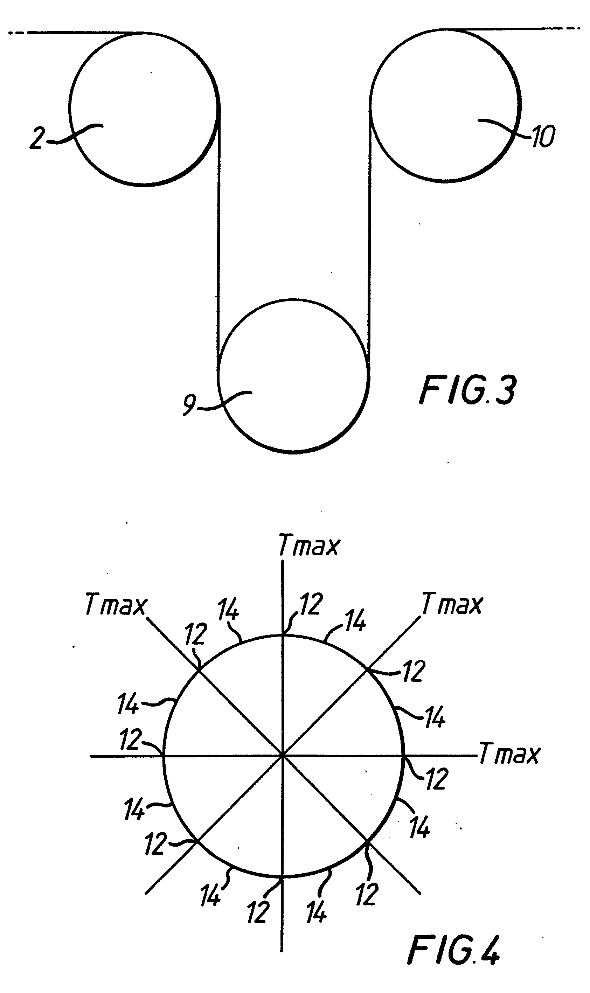

Figure 3 illustrates how it is possible to subject more than one line of the surface of the glass portion 3 to the maximum available strain T max by passing the fibre around another roller 9. This causes a part of the fibre diametrically opposite the line 7 to be subjected to the maximum strain and does so for about half a revolution of the roller. A third roller 10 is arranged such that the line 7 is subjected again to the same strain T max for about a quarter of a revolution and serves in addition to align the fibre 1 as it enters and leaves the set of three rollers 2, 9 and 10.

Thus it is possible to proof test a long length of glass fibre by passing it along a U-shaped path over two and under one of the set of three rollers as shown in Figure 3. Again the radius of the rollers is determined by the testing specification and the tension must be adequate to ensure good roller to fibre contact. However if only a single set of three rollers is used much of the surface of the fibre does not suffer the maximum strain and points on the surface along one pair of diametrically opposite lines pass through having suffered no strain. This effect can be reduced significantly by repeatedly passing the fibre through the set of rollers or preferably by mounting several sets of rollers so that the roller axes in the various sets are at angles to each other. For example four sets of rollers can be employed, the sets being spaced along a linear path extending at right angles to the roller axes. The sets of rollers are orientated so that, when viewed along the linear path, the axes of the rollers of the second set are at 45° in one sense to those of the first set, the axes of the rollers of the third set are at 45° in the opposite sense to those of the first set, and the axes of the rollers of the fourth set are at 90° to those of the first set. If a fibre is passed through four sets of rollers arranged in this manner the maximum strain T max is exerted along each of eight lines on the surface of the fibre. This is illustrated in Figure 4 in which the eight lines of maximum strain extend linearly along the surface of the glass portion 3 through the eight points 12. In this way not only is a much greater area of the glass surface fully strained but also the minimum strain that is experienced on the surface of the glass along the lines 14 in Figure 4, is kept as high as practicable and can be shown in this case to be greater than 90% of T max.

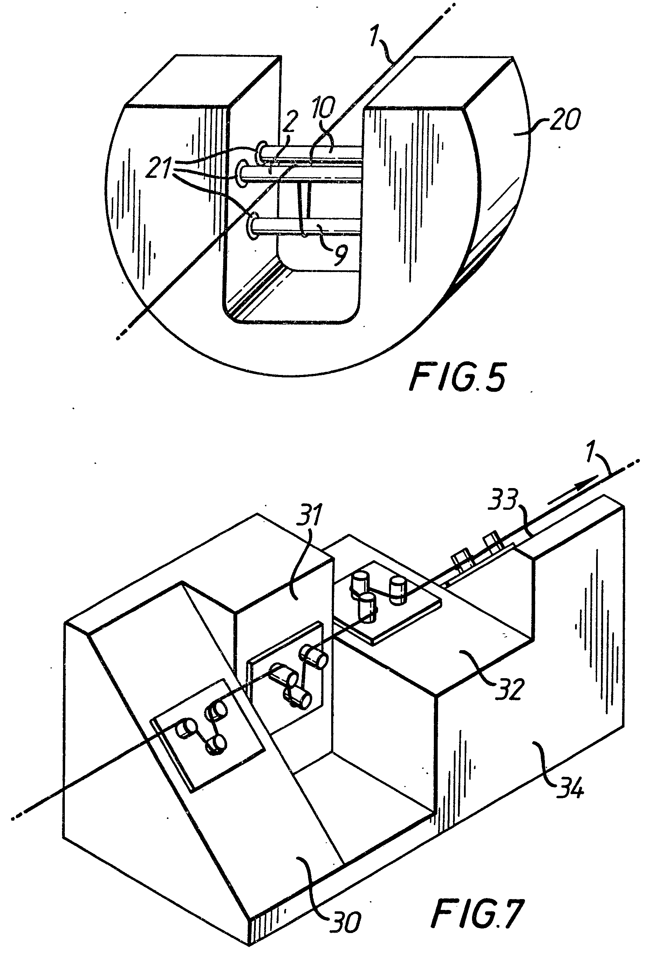

One way in which the rollers can be mounted is shown in Figure 5. A set of three stainless steel rollers 2, 9 and 10 is mounted as shown between the limbs of a generally U-shaped block 20. The ends of each roller are mounted in bearings 21 so that the rollers can rotate freely. The outer periphery of the block 20 is generally circular in cross-section.

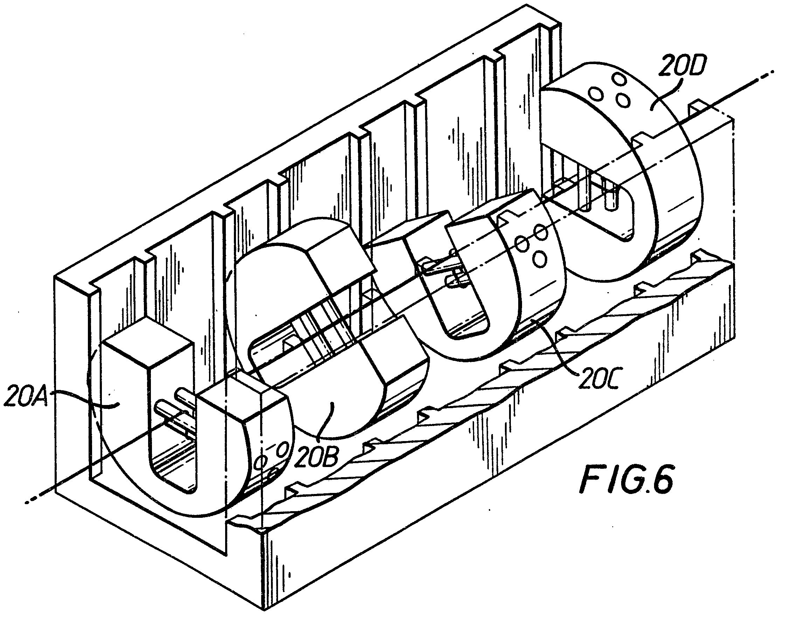

Figure 6 shows how four blocks 20A, 20B, 20C, 20D can be mounted in an open ended box to produce the regions of maximum tension illustrated in Figure 4. Each block is held within respective channels formed on the inner surface of the side-walls of the box. By virtue of the generally circular shape of the outer periphery of each block, the blocks can be rotated within the box so that they can be set to the correct relative orientation to produce the required regions of maximum tension illustrated in Figure 4.

Figure 7 illustrates an alternative way of mounting the rollers. The four sets of rollers are mounted on surfaces 30, 31, 32, 33 machined on a block 34. The surface 30 is inclined at 45° to the surface 31, the surface 32 at 90° to the surface 31 and the surface 33 at 90° to the surface 31. The rollers have the same relative orientations as in the arrangement of Figure 6 so that a fibre 1 drawn through the rollers is subjected to the strains illustrated in Figure 4. The arrangement of Figure 7 has the advantage that it is easier to thread the fibre around the rollers since they are mounted in bearings at one end only the other end being free. It should be noted that the rollers are free to rotate so that there is little or no possibility of frictional damage caused by fibre slippage on a roller.

The proof testing apparatus can be located at any convenient position. For example it could be located between the constant speed capstan and the take-off drum of the fibre pulling and coating apparatus described in "Fibre and Integrated Optics" Volume 2 Number 3—4, pages 267 to 285 (1979) and shown in Figure 2 thereof. This would allow optical fibres to be proof-tested as they are produced.

## Claims

1. A method of proof testing a glass fibre to detect fibres having a strength greater than a predetermined value, the method comprising moving the fibre (1) along a path in which it is bent over the surface of a roller (2) which is free to rotate, the roller (2) having a diameter such that tensile strain induced by bending in the surface of said optical fibre corresponds to the said predetermined value of strength, the fibres being drawn around the roller so that successive portions of said fibre are subjected to the tensile strain induced by bending.

2. A method of proof testing a glass fibre as claimed in claim 1 wherein said tensile strain is greater than 1%.

3. A method of proof testing a glass fibre as claimed in claim 1 or claim 2 wherein said fibre is passed through a set of three said rollers (2, 9, 10) which are arranged such that said fibre is bent in a first sense about a first roller (2), then in a sense opposite to said first sense about a second roller (9) and then in said first sense about a third roller (10).

4. A method as claimed in claim 3 wherein said rollers are arranged so that said tensile strain induced by bending is maintained through substantially 90° about said first roller (2), through substantially 180° about said second roller (9), and through substantially 90° about said third roller (10).

5. A method as claimed in claim 3 or claim 4 wherein said fibre is passed through n sets of three rollers, the rollers being orientated such that 2n axial lines on said surface of said fibre are subjected to a maximum value of said tensile strain induced by bending.

6. A method as claimed in claim 5 where n is equal to 4, and said 4 sets of rollers are located so that said 8 lines are equi-angularly spaced about the circumference of said fibres.

7. A method as claimed in any one of claims 1 to 6 wherein the fibre (1) is a glass optical fibre having a plastics sheath.

8. Apparatus for proof-testing glass fibres to detect glass fibres having a strength greater than a predetermined value, said apparatus comprising n sets of m rollers (2, 9, 10) where n and m are greater than 1, each roller being free to rotate and having a diameter such that tensile strain induced by bending in the surface of said optical fibre corresponds to the said predetermined value of strength, the rollers of each set of rollers being mounted in a respective one of n support means (20, 34), said n support means being arranged at predetermined angles to each other such that when a fibre is drawn through said sets of rollers maximum strain is induced in the fibre at positions substantially evenly distributed about the circumference of said fibre.

9. Apparatus as claimed in claim 8 where m equals 3 and said support means is a block 20, said rollers being mounted so that the axes of two rollers lie in a plane and are separated by a gap and the third roller is mounted with its axis parallel to, but out of said plane, and substantially midway between two planes which pass one through each axis of said two rollers in a direction normal to said one plane.

10. Apparatus as claimed in claim 8 or claim 9 wherein said support means is a U-shaped block 20 and the rollers (2, 9, 10) are located in the gap between the limbs of the block, each roller having a bearing means (21) in each limb of the block.

11. Apparatus as claimed in claim 10 including means for holding said blocks comprising an open ended box (22) having channels (23) on its inner walls for receiving and holding said U shaped blocks (20) in predetermined orientations.

12. Apparatus as claimed in claim 11 wherein the outer periphery of said U-shaped blocks (20) have a generally circular cross sectional shape with a segment removed, and a gap cut into the resultant shape centred on a diameter intersecting the segment chord at 90°, whereby said U-shaped blocks can be rotated within said channels (23) in said open ended box (22).

## Revendications

1. Procédé pour tester une fibre de verre, afin de détector les fibres dont la résistance est supérieure à une valeur prédéterminée, caractérisé par le fait qu'il consiste à déplacer la fibre (1) le long d'un trajet dans lequel elle est courbée sur la surface d'un galet (2) libre en rotation, le galet (2) ayant un diamètre tel que la contrainte de traction créée par la courbure de la surface de ladite fibre optique corresponde à ladite valeur prédéterminée de la résistance, les fibres étant tirées autour dudit galet de façon que des portions successives de ladite fibre soient soumises à la contrainte de traction créée par la courbure.

2. Procédé selon la revendication 1, dans lequel ladite contrainte de traction est supérieure à 1%.

3. Procédé selon l'une des revendications 1 et 2, dans lequel ladite fibre est acheminée à travers un ensemble de trois galets (2, 9, 10) disposés de telle sorte que la fibre est courbée dans une première direction autour d'un premier galet (2), puis dans une seconde direction opposée à ladite première direction autour d'un second galet (9), puis enfin dans ladite première direction autour d'un troisième galet (10).

4. Procédé selon la revendication 3, dans lequel lesdits galets sont disposés de façon que la contrainte de traction créée par la courbure soit maintenue sur sensiblement 90° autour dudit premier galet (2), sur sensiblement 180° autour dudit second galet (9) et sur sensiblement 90° autour dudit troisième galet (10).

5. Procédé selon l'une des revendications 3 et 4, dans lequel ladite fibre est acheminée à travers n ensembles de trois galets, les galets étant orientés de telle manière que la surface de ladite fibre soit soumise à une valeur maximale de ladite contrainte de traction créée par courbure selon 2n directions axiales.

6. Procédé selon la revendication 5, dans lequel n est égal à 4, et les 4 ensembles de galets disposés de telle manière que lesdites 8 directions soient régulièrement réparties angulairement autour de la circonférence de ladite fibre.

7. Procédé selon l'une des revendications 1 à 6, dans lequel la fibre (1) est une fibre de verre optique comportant une gaine de plastique.

8. Dispositif pour tester des fibres de verre afin de détecter les fibres dont la résistance est supérieure à une valeur prédéterminée, caractérisé par le fait qu'il comprend n ensembles de m galets (2, 9, 10), n et m étant supérieurs à 1, chacun des galets étant libre en rotation et ayant un diamètre tel que la contrainte de traction créée par la courbure de la surface de ladite fibre optique corresponde à ladite valeur prédéterminée de la résistance, les galets de chacun des ensembles de galets etant montés respectivement sur un parmi n moyens de support (20, 24), lesdits n moyens de support étant disposés les uns par rapport aux autres selon des angles prédéterminés, la dispositions étant telle que lorsqu'une fibre est tirée à travers lesdits ensembles de galets, la fibre est soumise à constrainte maximale dans des positions sensiblement régulièrement réparties autour de la circonférence de ladite fibre.

9. Dispositif selon la revendication 8, dans lequel m est égal à 3 et lesdits moyens formant support consistent en un bâti (20), les galets étant montés de façon que les axes de deux d'entre eux soient situés dans un même plan et séparés par un intervalle, le troisième galet étant monté de façon que son axe soit parallèle audit plan, mais non hors de celui-ci, et sensiblement à mi-distance entre deux plans passant respectivement par chacun des axes desdits deux galets et perpendiculaires audit premier plan.

10. Dispositif selon l'une des revendications 8 et 9, dans lequel lesdits moyens formant support consistent en un bâti en U (20), les galets (2, 9, 10) étant situés dans l'intervalle entre les branches du bâti, chaque galet comportant des moyens formant palier (21) dans chaque branche du bâti.

11. Dispositif selon la revendication 10, lequel comprend des moyens de maintien desdits bâtis qui comprennent un boîtier (22) à extrémités ouvertes comportant, sur ses parois intérieures, des rainures (23) destinées à recevoir et à maintenir dans des orientations prédéterminées lesdits bâtis en U (20).

12. Dispositif selon la revendication 11, dans lequel la périphérie extérieure desdits bâtis en U (20) présente, en section, une forme globalement circulaire dont un segment a été enlevé, un évidement ayant été pratiqué dans la forme résultante, centré sur un diamètre coupant la corde du segment à 90°, lesdits bâtis en U pouvant pivoter à l'intérieur desdites rainures (23) dans ledit boîtier (22) à extrémités ouvertes.

## Patentansprüche

1. Verfahren für die Belastungsprüfung einer Glasfaser zur Ermittlung von Fasern, deren Festigkeit größer als ein gegebener Wert ist, gekennzeichnet
— durch Bewegen der Faser (1) längs einer Bahn, in der sie um die Oberfläche einer frei drehbaren Walze (2) bewegt wird, deren Durchmesser so groß ist, daß die durch Biegung in der Oberfläche der optischen Faser erzeugte Zuglast dem gegebenen Festigkeitswert entspricht,
— wobei die Fasern so um die Walze gezogen werden, daß aufeinanderfolgende Teile der Faser der durch die Biegung erzeugten Zuglast ausgesetzt werden.

2. Verfahren für die Belastungsprüfung einer Glasfaser nach Anspruch 1, dadurch gekennzeichnet,
— daß die Zuglast größer als 1% ist.

3. Verfahren für die Belastungsprüfung einer Glasfaser nach Anspruch 1 oder 2, dadurch gekennzeichnet,
— daß die Faser durch einen Satz von drei Walzen (2, 9, 10) bewegt wird, die so angeordnet sind, daß die Faser um eine erste Walze (2) in einem ersten Sinn, um eine zweite Walze (9) in einem zum ersten Sinn entgegengesetzten Sinn und um eine dritte Walze (10) im ersten Sinn gezogen wird.

4. Verfahren nach Anspruch 3, dadurch gekennzeichnet,
— daß die Walzen so angeordnet sind, daß die durch Biegung erzeugte Zuglast um die erste Walze (2) auf im wesentlichen 90°, um die zweite Walze (9) auf im wesentlichen 180° und um die dritte Walze (10) auf im wesentlichen 90° gehalten wird.

5. Verfahren nach Anspruch 3 oder 4, dadurch gekennzeichnet,
— daß die Faser durch n Sätze von drei Walzen bewegt wird,
— wobei die Walzen so ausgerichtet sind, daß 2n axiale Linien auf der Oberfläche der Faser einem Maximalwert der durch Biegung erzeugten Zuglast ausgesetzt sind.

6. Verfahren nach Anspruch 5, dadurch gekennzeichnet,
— daß n gleich 4 ist und
— daß vier Sätze von Walzen so angeordnet sind, daß die acht Linien um den Umfang der Faser gleiche Winkelabstände haben.

7. Verfahren nach einem der Ansprüche 1 bis 6, dadurch gekennzeichnet,
— daß die Faser (1) eine optische Glasfaser mit einer Kunststoffhülle ist.

8. Vorrichtung für die Belastungsprüfung von Glasfasern zur Ermittlung von Glasfasern, deren Zugfestigkeit größer als ein gegebener Wert ist, gekennzeichnet
— durch n Sätze von m Walzen (2, 9, 10),
— wobei n und m größer als 1 sind,
— wobei jede Walze friedrehbar ist und einen solchen Durchmesser hat, daß die durch Biegung in der Oberfläche der optischen Faser erzeugte Zuglast dem gegebenen Festigkeitswert entspricht,
— wobei die Walzen jedes Satzes von Walzen in einer entsprechenden von n Trageinrichtungen (20, 34) gelagert sind.
— wobei die n Trageinrichtung unter gegebenen Winkeln zueinander so angeordnet sind, daß beim Ziehen einer Faser durch die Sätze von Walzen in der Faser eine maximale Belastung an Stellen erzeugt wird, die im wesentlichen gleichmäßig um den Umfang der Faser verteilt sind.

9. Vorrichtung nach Anspruch 8, dadurch gekennzeichnet,
— daß m gleich 3 ist und
— daß die Trageinrichtung ein Block (20) ist,
— wobei die Walze so gelagert sind,
— daß die Achsen zweier Walzen in einer Ebene liegen und durch einen Spalt getrennt sind und daß die Achse der dritten Walze parallel zu und außerhalb dieser Ebene sowie im wesentlichen in der Mitte zwischen zwei Ebenen angeordnet ist, die sich jeweils durch eine der beiden Achsen senkrecht zur einen Ebene erstrecken.

10. Vorrichtung nach Anspruch 8 oder 9, dadurch gekennzeichnet,
— daß die Trageinrichtung ein U-förmiger Block (20) ist und
— daß die Walzen (2, 9, 10) im Spalt zwischen den Schenkeln des Blocks angeordnet sind,
— wobei jede Walze in jedem Schenkel des Blocks eine Lageranordnung (21) aufweist.

11. Vorrichtung nach Anspruch 10 gekennzeichnet
— durch eine Einrichtung zum Halten der Blöcke mit einem offen endenden Kasten (22) einschließlich Nuten (23) auf dessen Innenwänden zum Aufnehmen und Halten der U-förmigen Blöcke (20) in gegebenen Ausrichtungen.

12. Vorrichtung nach Anspruch 11, dadurch gekennzeichnet,
— daß der Außenumfang der U-förmigen Blöcke (20) aufweist: einem im allgemeinen kreisförmigen Querschnitt, wobei ein Segment entfernt ist, und einen Spalt, der in der verbleibenden Gestalt eingeschnitten und auf einem die Segmentsehne unter 90° schneidenden Durchmesser zentriert ist, wodurch die U-förmigen Blöcke innerhalb der Nuten (23) im offen endenden Kasten (22) gedreht werden können.

0 025 646

FIG.1

FIG.2

**FIG.3**

**FIG.4**

FIG.5

FIG.7

3

FIG.6